# EUROPÄISCHE PATENTSCHRIFT

(11) **EP 0 845 475 B1**
(45) Veröffentlichungstag und Bekanntmachung des Hinweises auf die Patenterteilung: **09.03.2005**
(21) Anmeldenummer: 97119835.3
(22) Anmeldetag: 13.11.1997
(51) Int. Cl.: C07H 3/04, C07H 3/06, A61K 31/70

(54) **Inositolglykane mit insulinartiger Wirkung**
Inositolglycans with insular activity
Glycans d'inositol avec une activité insulinique

(30) Priorität: 28.11.1996 DE 19649350
(43) Veröffentlichungstag der Anmeldung: 03.06.1998
(73) Patentinhaber: Aventis Pharma Deutschland GmbH, 65929 Frankfurt am Main (DE)
(72) Erfinder: Frick, Wendelin, Dr., 65510 Hünstetten-Beuerbach (DE); Müller, Günter, Dr., 65843 Sulzbach (DE)

(56) Entgegenhaltungen:
- EP-A- 0 532 915
- EP-A- 0 545 198
- WO-A-96/14075
- US-A- 4 906 468
- CHEMICAL ABSTRACTS, vol. 123, no. 7, 1995 Columbus, Ohio, US; abstract no. 83943s, T.MURAGATA ET AL.: "Preparation of Physiologically Active Inositol Glycans." Seite 1105; Spalte 2; XP002058260 & JP 06 293 790 A (KYOWA HAKKO KOGYO KK)
- A.ZAPATA ET AL.: "Building Blocks for the Synthesis of the Glycosyl-myo-Inositols Involved in the Insulin Intracellular Signalling Process." CARBOHYDRATE RESEARCH, Bd. 234, 9.Oktober 1992, Seiten 93-102, XP002058259
- Frick et al: (1998) Biochemistry 37, 13421-13436 XP000946196

## Beschreibung

Die Erfindung betrifft Inositolglykane mit insulinartiger Wirkung, die sich zur Behandlung des Diabetes mellitus eignen.

Es ist bekannt, daß die metabolische Wirkung von Insulin auch die Bildung von niedermolekularen Verbindungen bewirkt, die auch insulinartige Wirkung besitzen (US 4,446,064). Es sind schon eine Reihe von Inositolglykanverbindungen vorgeschlagen worden, die insulinartige Wirkung zeigen (WO 96/14075, JP 6/293790, JP 4/120089)

Der Diabetes Typ II, nicht insulinabhängiger Diabetes, geht einher mit einer Insulinresistenz der peripheren Gewebe, wie Muskel- oder Fettgewebe. Die dadurch reduzierte Glucoseverwertung wird verursacht durch fehlende Insulinstimulation des Glucosetransports und nachfolgender metabolischer Prozesse.

In dem Bestreben weitere wirksame Verbindungen mit insulinartiger Wirkung zu finden, wurde nun gefunden, daß die erfindungsgemäßen Verbindungen in vitro insulinartige Wirkung zeigen, eine gute Serumstabilität aufweisen und auch an insulinresistenten Geweben insulinartige Wirkung zeigen und sich so zur Behandlung von Diabetes mellitus eignen.

Die Erfindung betrifft daher Inositolglykane mit insulinartiger Wirkung der Formel I

A - Z - R (I)

und/oder physiologisch verträgliche Salze der Verbindung der Formel I und/oder stereoisomere Formen der Verbindung der Formel I, wobei
- A: für
1) H - P(O)(OH) -,
2) S(O)₂(OR¹) - oder
3) NH₂ - C(O) - steht,
- Z: für
1 ) 2 bis 6 Zuckerreste aus der Gruppe
   1.1 Mannose,
   1.2 Glucose,
   1.3 Gluconsäure,
   1.4 Galactonsäure,
   1.5 Mannonsäure,
   1.6 Glucosamin,
   1.7 Fructose oder
   1.8 Galaktose oder
2) 2 bis 6 Zuckerreste aus der Gruppe definiert unter Z 1.1 bis 1.8 stammen und ein- bis sechsfach unabhängig voneinander substituiert sind durch
   2.1 Methyl,
   2.2 Mannose,
   2.3 Glucosamin,
   2.4 Dimannose oder
   2.5 Mannose-Glucosamin und die glykosidische Bindung der beiden Zucker Mannose und Glucosamin zwischen den C-Atomen 1-3, 1-2 oder 1-6 der beiden Zucker liegt,
steht
und
- R: für
1) Inositol,
2) Inositolphosphat,
3) Inositolthiophosphat,
4) Inositofcyclothiophosphat oder
5) Inositolcyclophosphat steht.
Insbesondere bevorzugt sind Verbindungen der Formel I, die dadurch gekennzeichnet sind, daß
- A: für H - P(O)(OH) - steht,
- Z für: 1) 2 bis 4 Zuckerreste aus der Gruppe
   1.1 Mannose oder
   1.2 Glucosamin stammen oder
2) 2 bis 4 Zuckerreste aus der Gruppe
   2.1 Mannose oder
   2.2 Glucosamin stammen, einfach substituiert durch Mannose steht und
- R: für
1) Inositol,
2) Inositolphosphat oder
3) Inositolcyclophosphat steht.

Unter Zuckerresten werden Verbindungen verstanden, die sich von Aldosen und Ketosen mit 3 bis 7 Kohlenstoffatomen ableiten, die der D- oder L-Reihe angehören können; dazu gehören auch Aminozucker oder Uronsäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure oder Mannonsäure.

Mit Dizucker sind Saccharide gemeint, die aus zwei Zuckereinheiten bestehen. Tri-, Tetra-, Penta- oder Hexazucker entstehen durch acetalartige Bindung von 3 bis 6 Zuckern. Die Bindungen können dabei in der α- oder β-Form auftreten. Die Bindungen zwischen den Zuckern erfolgen bevorzugt über C-Atom 1 und C-Atom 6, C-Atom 1 und C-Atom 2 sowie C-Atom 1 und C-Atom 4 der jeweiligen Zucker. Die α-Form der Bindung zwischen den Zuckern ist bevorzugt.

Die Bindung von A an Z erfolgt beispielsweise über eines der Sauerstoffatome von Z oder über eines der Kohlenstoffatome von Z, bevorzugt über das Kohlenstoffatom der CH₂-Gruppe von Z. Die Bindung der Reste für A 10) bis 12) erfolgt bevorzugt über ein Kohlenstoffatom von Z, die anderen Bindungen von A erfolgen bevorzugt über ein Sauerstoffatom von Z.

Die Bindung von R an Z erfolgt analog zu den Bindungen der Di-, Tri-, Tetra-, Penta- oder Hexazucker. Ferner kann die Bindung von R an Z auch ein- oder mehrfach durch - CH₂ - oder - S - ersetzt sein.

Wenn der Zucker substituiert ist, so erfolgt die Substitution bevorzugt am Wasserstoffatom einer OH-Gruppe des Zuckers.

Unter dem Begriff insulinresistentes Gewebe werden beispielsweise Rattenfettzellen verstanden, die keinen Insulinrezeptor mehr besitzen.

Die erfindungsgemäßen Verbindungen können eine oder mehrere Phosphatgruppen enthalten, die auch noch mit einer Phosphatschutzgruppe derivatisiert sein können. Phosphatschutzgruppen sind beispielsweise Phenyl, Benzyl oder Hydroxypropylnitril (Houben Weyl, Methoden der Organischen Chemie, Band 12/1 oder Band 12/2; Teilheimer, Synthetic Methods of Organic Chemistry, Vol 45).

Unter physiologisch verträglichen Salzen der Verbindung der Formel I sind insbesondere pharmazeutisch verwendbare oder nicht-toxische Salze zu verstehen. Solche Salze werden z.B. von Verbindungen der Formel I, welche saure Gruppen, z.B. Phosphate oder Sulfate, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin. Verbindungen der Formel I, welche basische Gruppen, z.B. eine Aminogruppe enthalten, bilden mit anorganischen Säuren, wie z. B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure Salze. Verbindungen, in denen basische und saure Gruppen in gleicher Zahl vorliegen, bilden innere Salze und sind auf eine dritte Salzkomponente nicht angewiesen.

Die Erfindung betrifft weiterhin ein Verfahren zur Herstellung der Verbindung der Formel I, das dadurch gekennzeichnet ist, daß man das Inositolglykan stufenweise aus geschützten Zucker- und Inositol-Vorstufen aufbaut, anschließend den Rest A anfügt und von der erhaltenen Verbindung eine oder mehrere zum Schutz anderer Funktionen temporär eingeführte Schutzgruppen abspaltet und die so gewonnene Verbindung der Formel I gegebenenfalls in ihr physiologisch verträgliches Salz überführt.

Die Synthese der Di- bis Mehrfachzucker erfolgt nach bekannten Verfahren (H. Paulsen, Angew. Chem. Int. Ed. 21 (1982) S. 155). Bevorzugt wird die Trichloracetimidat Methode zur Synthese von Oligosacchariden angewendet (R. R. Schmidt, Angew. Chem. Int. Ed. 25 (1986) 212 - 235; T. Ogawa, Tetrahedron Lett. 31 (1990) 2439 - 2442).

Die Synthese der Phosphate erfolgt mit Hilfe der H-Phosphat- und der Phosphoramidit-Methode. (W. Bannwath et al., Helvetica Chemica Acta, 70 (1987), Seiten 175-186; L.A. Slotin, Synthesis (1977), Seiten 737-752)

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden-, Cyclohexyliden- oder Isopropylidenschutzgruppen.

Die Verbindungen der Formel I und deren physiologisch verträgliche Salze dienen in erster Linie als Wirkstoffe für pharmazeutische Zubereitungen zur Behandlung des Diabetes mellitus oder nicht insulinabhängiger Diabetes.

Erfindungsgegenstand ist daher auch eine pharmazeutische Zubereitung, die durch einen Gehalt an mindestens einer Verbindung der Formel I und/oder mindestens einem von dessen physiologisch verträglichen Salzen in gelöster, amorpher und/oder kristalliner - vorzugsweise in amorpher und/oder kristalliner Form gekennzeichnet ist.

Die pharmazeutische Zubereitung ist vorzugsweise eine Lösung oder Suspension zu Injektionszwecken mit einem pH-Wert von etwa 3,0 bis 9,0, vorzugsweise von etwa 5,0 bis 8,5, welche ein geeignetes Isotoniemittel, ein geeignetes Konservierungsmittel und gegebenenfalls einen geeigneten Puffer, sowie gegebenenfalls auch ein Depotprinzip, alles in steriler wäßriger Lösung oder Suspension, enthält. Die Gesamtheit der Zubereitungsbestandteile außer dem Wirkstoff bildet den Zubereitungsträger. Geeignete Isotoniemittel sind z.B. Glycerin, Glucose, Mannit, NaCl, Calcium- oder Magnesium-Verbindungen wie z.B. CaCl₂ oder MgCl₂. Geeignete Konservierungsmittel sind z.B. Phenol, m-Kresol, Benzylalkohol und/oder p-Hydroxybenzoesäureester.

Als Puffersubstanzen, insbesondere zur Einstellung eines pH-Wertes von etwa 5,0 bis 8,5 können z.B. Natriumacetat, Natriumcitrat oder Natriumphosphat verwendet werden. Ansonsten sind zur Einstellung des pH-Wertes auch physiologisch unbedenkliche verdünnte Säuren (typischerweise HCl) bzw. Laugen (typischerweise NaOH) geeignet.

Zwecks Variation des Wirkungsprofils der erfindungsgemäßen Zubereitung können auch modifizierte (vgl. EP-B 132 769 und EP-B 132 770) und/oder unmodifizierte Insuline, vorzugsweise Rinder-, Schweine- oder Humaninsulin, insbesondere Humaninsulin, zugemischt werden.

Die pharmazeutische Zubereitung wird dadurch hergestellt, daß man mindestens eine Verbindung der Formel I und/oder mindestens eines von dessen physiologisch verträglichen Salzen, gegebenenfalls zusammen mit modifizierten und/oder unmodifizierten Insulin(derivat)en, mit einem physiologisch unbedenklichen Träger sowie gegebenenfalls mit geeigneten Zusatz- und Hilfsstoffen in eine geeignete Darreichungsform bringt.

Die Erfindung wird nun durch die folgenden Beispiele näher erläutert.

### Beispiel 1:

### Herstellung der Verbindung B

Der Syntheseverlauf ist dem Formelschema am Ende von Beispiel 1 zu entnehmen.

### Synthese von Verbindung 3

60 g (94 mmol) **1** (T. G. Mayer, B. Kratzer, R. R. Schmidt, Angew. Chem. 106 (1994) 2289- 93) und 21,2 g (42,4 mmol) **2** (A. Termin, R. R. Schmidt, Liebigs Ann. Chem. (1989) 789- 795) werden in 200 ml trockenem Methylenchlorid und 400 ml trockenem n-Heptan gelöst. Nach Zugabe von 70 g getrocknetem Molsieb (0,4 nm) wird 15 Minuten bei Raumtemperatur gerührt. Dann werden 5 ml 0,05 M Trimethylsilyl-trifluormethansulfonsäure in Methylenchlorid (in den nachfolgenden Vorschriften als Katalysator-Lösung bezeichnet) zugegeben. Nach 15 Minuten gibt man 300 ml n-Heptan/ Essigester (1:1) zu und filtriert über Kieselgel. Es wird mit n-Heptan/Essigester (1:1) nachgewaschen und dann eingeengt. Nach Reinigung mittels Flashchromatographie erhält man 41,0 g (99%) **3** als farbloses Öl. DC: n-Heptan/Essigester (1:1), R_{f} = 0,6, MS: (M + Li)⁺ = 981,1, berechnet C₅₅H₆₇N₃O₁₁Si, M= 974,21.

### Synthese von Imidat 4

41 g (42,0 mmol) 3 werden in 400 ml Tetrahydrofuran (THF) und 9 ml Essigsäure gelöst. Nach Zugabe von 70 ml 1 M TBAF/THF- Lösung läßt man 8 Stunden bei Raumtemperatur stehen. Die Essigsäure wird durch ausfrieren (16h bei -30°C) entfernt und das Filtrat nach Einengen mittels Flashchromatographie gereinigt. Ausbeute 34,1 g (94%) entschützte Verbindung. Diese wird in 300 ml trockenem Methylenchlorid gelöst. Nach Zugabe von 50 ml Trichloracetonitril und 20 g Kaliumcarbonat läßt man 4 Stunden bei Raumtemperatur rühren. Es wird über Kieselgel filtriert, mit n-Heptan/Essigester (1:1) nachgewaschen und eingeengt. Rohausbeute: 42,1g. DC: n-Heptan/Essigester (2:1), R_{f} = 0,5. ¹H- NMR (CDCl₃): charakteristische Signale für Imidat; σ = 8,78, für NH und 5,63 für das Anomere (β-Imidat).

### Synthese von Trisaccharid 6

33,2g (33,0 mmol) **4** und 13,3 g (25,0 mmol) **5** (R. Aneja, S. G. Aneja, A. Parra, Tetrahedron, Asymmetry 6 (1995) 17- 18; C. J. J. Elie, R. Verduyn, C. E. Dreef, D. M. Braunts, G. A. van der Marel, J. H van Boom, Tetrahedron, 46 (1990) 8243- 54) werden in 120 ml trockenem Methylenchlorid und 360 ml trockenem n-Heptan gelöst. Nach Zugabe von 100 g Molsieb wird 15 Minuten bei Raumtemperatur gerührt. Es wird unter Argon auf -20°C abgekühlt und dann mit 20 ml Katalysatorlösung versetzt. Nach 30 Minuten läßt man auf Raumtemperatur auftauen. Zur Aufarbeitung wird über Kieselgel filtriert und mit n- Heptan/Essigester (1:1) nachgewaschen. Es wird eingeengt und das Rohprodukt (46,2 g) in 150 ml Methylenchlorid gelöst. Nach Zugabe von 400 ml Methanol und 15 ml 1 M NaOMe/MeOH-Lösung läßt man 16 Stunden bei Raumtemperatur stehen. Die Lösung wird mit 1 ml Wasser versetzt und eingeengt. Das erhaltene Öl wird mit 50 ml Essigester gelöst, mit 200 ml n-Heptan/Essigester (1:1) verdünnt und über Kieselgel filtriert. Nach dem Einengen wird mit Flashchromatographie gereinigt. Ausbeute 32,5 g (97 %) weißer Schaum als Anomerengemisch. DC: n-Heptan/Essigester (2:1), R_{f} =0,5. MS: (M +Li)⁺ = 1336,7; berechnet C₈₀H₈₇N₃O₁₅, M = 1330,59.

### Synthese von Trisaccharid 7

Das Anomerengemisch **6** kann nur als Derivat **7** leicht chromatographisch getrennt werden. 32,4 g (24,4 mmol) **6** werden in 200 ml Methylenchlorid gelöst. Nach Zugabe von 500 ml 0,5 M HCl/MeOH (aus 17,5 ml AcCl in 500 ml MeOH) und 20 ml Ethylenglycol läßt man 17 Stunden bei Raumtemperatur stehen. Nach dem Einengen wird mit Flashchromatographie gereinigt. Das erhaltene Produkt (26,9 g, 88 %) wird in 300 ml Methylenchlorid gelöst. Man gibt 3,0 g Imidazol und 4,6 g TBDMSCI zu. Nach 16 Stunden bei Raumtemperatur wird mit 500 ml n-Heptan/Essigester (2:1) verdünnt und über Kieselgel filtriert. Es wird mit n-Heptan/Essigester (2:1) nachgewaschen und eingeengt. Das erhaltene Rohprodukt reinigt man mit Flashchromatographie. Ausbeute 21,1 g (72 %) **7** und 6,3 g (22 %) alpha Produkt. DC: n-Heptan/Essigester (2:1), R_{f} =0,5 für **7** und R_{f} =0,3 für das alpha Produkt. MS: (M +Li)⁺ = 1370,6; berechnet C₈₀H₉₃N₃O₁₅Si, M = 1364,71.

### Synthese von Trisaccharid 8

21,2 g (15.5 mmol) **7** werden in 60 ml Methylenchlorid und 180 ml Dimethoxypropan gelöst. Man gibt 250 mg TsOH zu und läßt 1 Stunde bei Raumtemperatur stehen. Nach Zugabe von 2 ml Triethylamin wird eingeengt und man erhält 23,1 g Rohprodukt. Dieses wird in 150 ml THF gelöst und mit 30 ml 1 M TBAF /THF-Lösung versetzt. Nach 16 Stunden wird eingeengt und mit Flashchromatographie gereinigt. Ausbeute: 20,0 g (99 %) **8** als weißer Schaum. DC: n-Heptan/Essigester (2:1), R_{f}= 0,4. MS: (M + Li)⁺ = 1296,7; berechnet C₇₇H₈₃N₃O₁₅, M= 1290,51.

### Synthese von Tetrasaccharid 10

20,0 g (15,4 mmol) **8** und 15,0 g (23,5 mmol) **9** (T. G. Mayer, B. Kratzer, R. R. Schmidt, Angew. Chem. 106 (1994) 2289- 93) werden analog der Vorschrift für Verbindung **6** umgesetzt und man erhält 20,3 g (76 %) Tetrasaccharid **10** als weißen Schaum. DC: n-Heptan/Essigester (2:1), R_{f}= 0,6, MS: (M + Li)⁺ = 1770, berechnet C₁₀₇H₁₁₅N₃O₂₀, M= 1763,09.

### Synthese von Pentasaccharid 12

20,3 g (11,8 mmol) **10** und 12,0 g (20,3 mmol) **11**(T. G. Mayer, B. Kratzer, R. R. Schmidt, Angew. Chem. 106 (1994) 2289- 93) werden analog der Vorschrift für Verbindung **6** umgesetzt und man erhält 19,4 g (80 %) deacyliertes Produkt. Dieses Produkt wird in 200 ml Methylenchlorid gelöst und mit 3,4 g (50,0 mmol) Imidazol versetzt. Man gibt 6,0 g (40,0 mmol) TBDMSCI zu und rührt 15 Stunden bei Raumtemperatur. Nach Zugabe von 5 ml Methanol läßt man 10 Minuten stehen, verdünnt dann mit 200 ml n-Heptan/Essigester (1:1) und filtriert über Kieselgel. Es wird noch mit 200 ml n-Heptan/Essigester (1:1) nachgewaschen, eingeengt und mittels Flashchromatographie gereinigt. Ausbeute: 19,4 g (95 %) **12** als weißer Schaum. DC: n-Heptan/Essigester (2:1), R_{f}= 0,7. MS: (M + Li)⁺ = 2226; berechnet C₁₃₃H₁₅₁N₃O₂₅Si, M= 2219,75

### Synthese von Hexasaccharid 14

19,4 g (8,9 mmol) **12** und 14,0 g (20,0 mmol) **13** (T. G. Mayer, B. Kratzer, R. R. Schmidt, Angew. Chem. 106 (1994) 2289- 93) werden in 100 ml trockenem Methylenchlorid und 300 ml trockenem n-Heptan gelöst. Nach Zugabe von 40 g Molsieb (0,4 nm) wird 15 Minuten bei Raumtemperatur gerührt. Es werden 10 ml Katalysatorlösung zugegeben und weitere 15 Minuten gerührt. Zur Aufarbeitung wird über Kieselgel filtriert und mit n-Heptan/Essigester (1:1) nachgewaschen. Es wird eingeengt und man erhält 33 g Rohprodukt. Dieses wird in 200 ml Methylenchlorid gelöst und mit 500 ml 0,5 M HCl in Methanol versetzt. Nach 2 Stunden bei Raumtemperatur wird eingeengt und mehrmals mit Methylenchlorid eingeengt. Das erhaltene Zwischenprodukt wird im 200 ml Methylenchlorid gelöst und mit 3,4 g (50 mmol) Imidazol und 6,0 g (40 mmol) TBDMSCI versetzt. Nach 16 Stunden (h), wird analog der Verbindung **12** aufgearbeitet. Ausbeute 20,2 g (85 %) **14** als weißer Schaum. DC: n-Heptan/Essigester (2:1), R_{f}= 0,3. MS: (M+Li)⁺= 2669; berechnet C₁₆₁H₁₇₇N₃O₃₀Si, M= 2662,26

### Synthese von Verbindung 15

30,0 g Triazol werden in 800 ml trockenem THF gelöst. Bei 10°C werden 13,5 ml Phosphoroxychlorid zugetropft. Danach tropft man 60 ml Triethylamin zu und rührt 15 Minuten bei Raumtemperatur. Der Niederschlag wird filtriert und mit wenig trockenem THF gewaschen. Das Filtrat wird zu 19,1 g (7,2 mmol) **14** gegeben. Die Lösung wird auf 100 ml konzentriert. Nach 15 Minuten wird mit 500 ml Essigester verdünnt und zweimal mit 100 ml Wasser gewaschen. Die organische Phase wird über Magnesiumsulfat getrocknet, filtriert und eingeengt. Nach Flashchromatographie erhält man 19,0 g (97 %) cyclisches Phosphatderivat als weißen Schaum. DC: MethylenchloridlMethanol/33% NH₃ (100/7/1), R_{f}= 0,3. MS: (M+2Li-H)⁺ = 2737; berechnet C₁₆₁H₁₇₄N₃O₃₂PSi, M= 2724,22. Das cyclische Phosphat wird in 350 ml THF gelöst und 100 ml TBAF (1M in THF) zugegeben. Nach 20 Stunden wird eingeengt und der Rückstand mit Flashchromatographie gereinigt. Ausbeute 18,1 g (99%) **15** als weißer Schaum. DC: Methylenchlorid/Methanol/33% NH₃ (100/7/1), R_{f}= 0,3 ( läuft gleich wie das Edukt). MS: (M+2Li-H)⁺ = 2622; berechnet C₁₅₅H₁₆₂N₃O₃₂P, M= 2609,96.

### Synthese von Verbindung 16

14 g Phosphorigesäure wird viermal mit Pyridin eingeengt und dann in 200 ml trockenem Pyridin aufgenommen. Bei 10°C tropft man 16 ml Pivaloylchlorid zu. Diese Reaktionslösung läßt man 15 Minuten bei Raumtemperatur stehen. 18,1 g (6,9 mmol) **15** werden in die oben beschriebene Reaktionslösung eingetragen. Nach 1 Stunde wird mit 200 ml Toluol und 150 ml Methylenchlorid/Methanol/33% NH₃ (30/10/3) verdünnt. Nach dem Einengen wird noch dreimal mit Toluol restliches Pyridin herausdestilliert. Der Rückstand wird in 200 ml Methylenchlorid/Methanol (20:1) suspendiert. Die nicht löslichen Bestandteile werden filtriert und zweimal mit 50 ml Methylenchlorid/Methanol (20:1) gewaschen. Das Filtrat wird eingeengt und mit Flashchromatographie gereinigt. Ausbeute 16,9 g (91%) geschütztes Endprodukt. DC: Methylenchlorid/Methanoll33% NH₃ (100/7/1 ), R_{f} = 0,25. MS: (M+3Li-2H)⁺ = 2691; berechnet C₁₅₅H₁₆₃N₃O₃₄P₂, M = 2673,94. Zum Entschützen werden 600 ml Ammoniak bei -78°C kondensiert. 4,7 g (204 mmol) Natrium werden darin gelöst. Diese Lösung wird mit 300 ml trockenem THF verdünnt und dann werden 16,9 g (6,3 mmol) geschütztes Endprodukt gelöst in 100 ml trockenem THF langsam bei -78°C Reaktionstemperatur zugetropft. Nach 15 Minuten Reaktionszeit (blaue Farbe darf nicht verschwinden) wird vorsichtig mit 10 g Ammoniumchlorid versetzt. Wenn die blaue Farbe verschwindet wird vorsichtig mit 100 ml Wasser und 300 ml Methanol verdünnt. Man läßt auftauen und engt dann auf rund 150 ml ein. Diese Lösung wird mit 2 l Methylenchlorid/Methanol/33% NH₃ (3/3/1) verdünnt und auf eine Flashkieselgelsäule (700 ml Kieselgel) gegeben. Es wird mit 3 l Methylenchlorid/Methanol/33% NH₃ (3/3/2) dann mit 3 l (3/3.5/3) eluiert. Das Produkt eluiert, wenn man dann mit n-Butanol/Ethanol/Wässer/33% NH₃ (2/2/2/1) chromatographiert. Ausbeute 5,5 g (78 %) **16** als weißer Feststoff. DC: (2/2/2/1), R_{f} = 0,4. MS: (M+H)⁺= 1116,5; berechnet C₃₆H₆₃NO₃₄P₂, M= 1115,83. ³¹P- NMR (D₂O) σ = 16,3 PPM für cyclisches Phosphat und 7,9 für H- Phosphat.

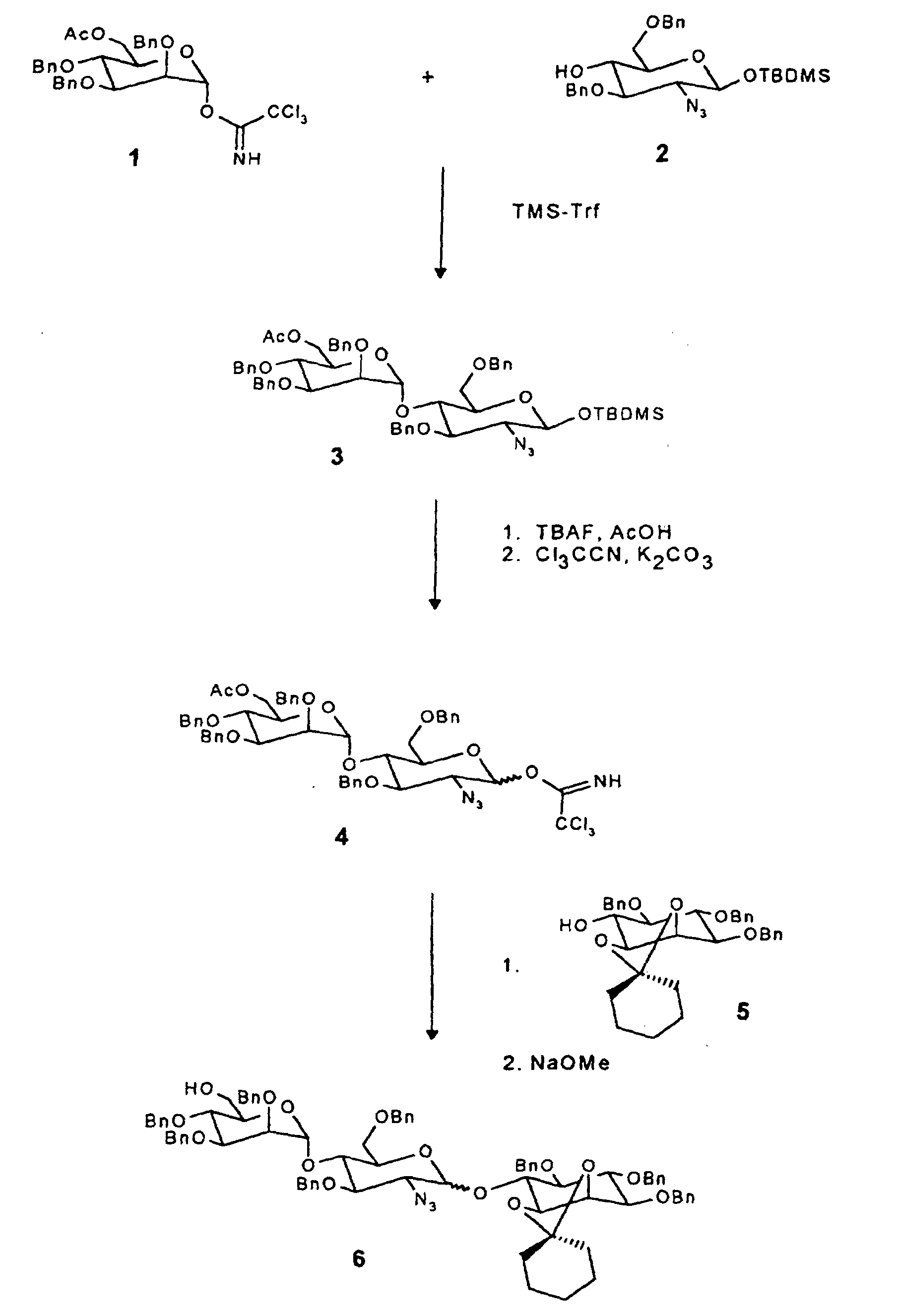

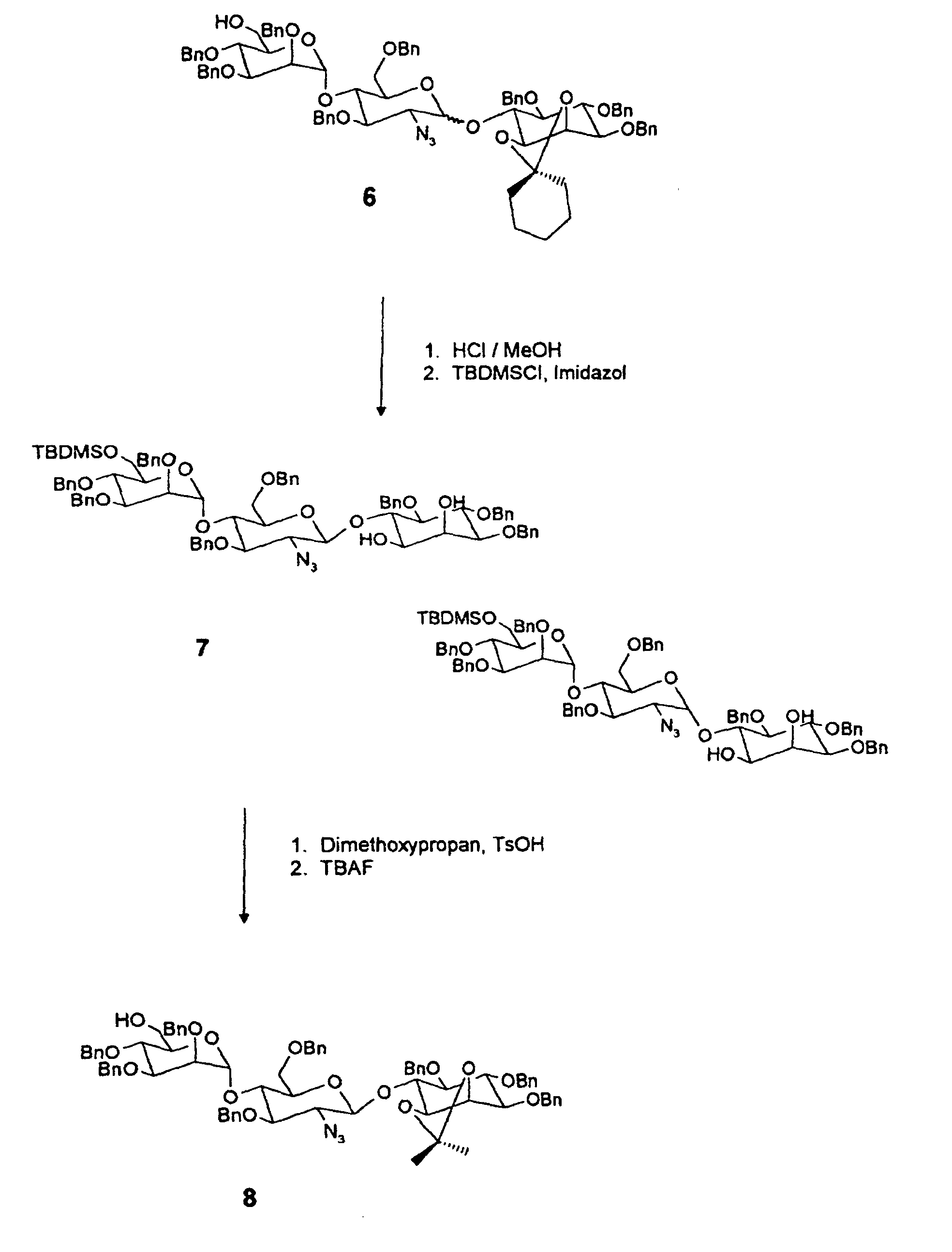

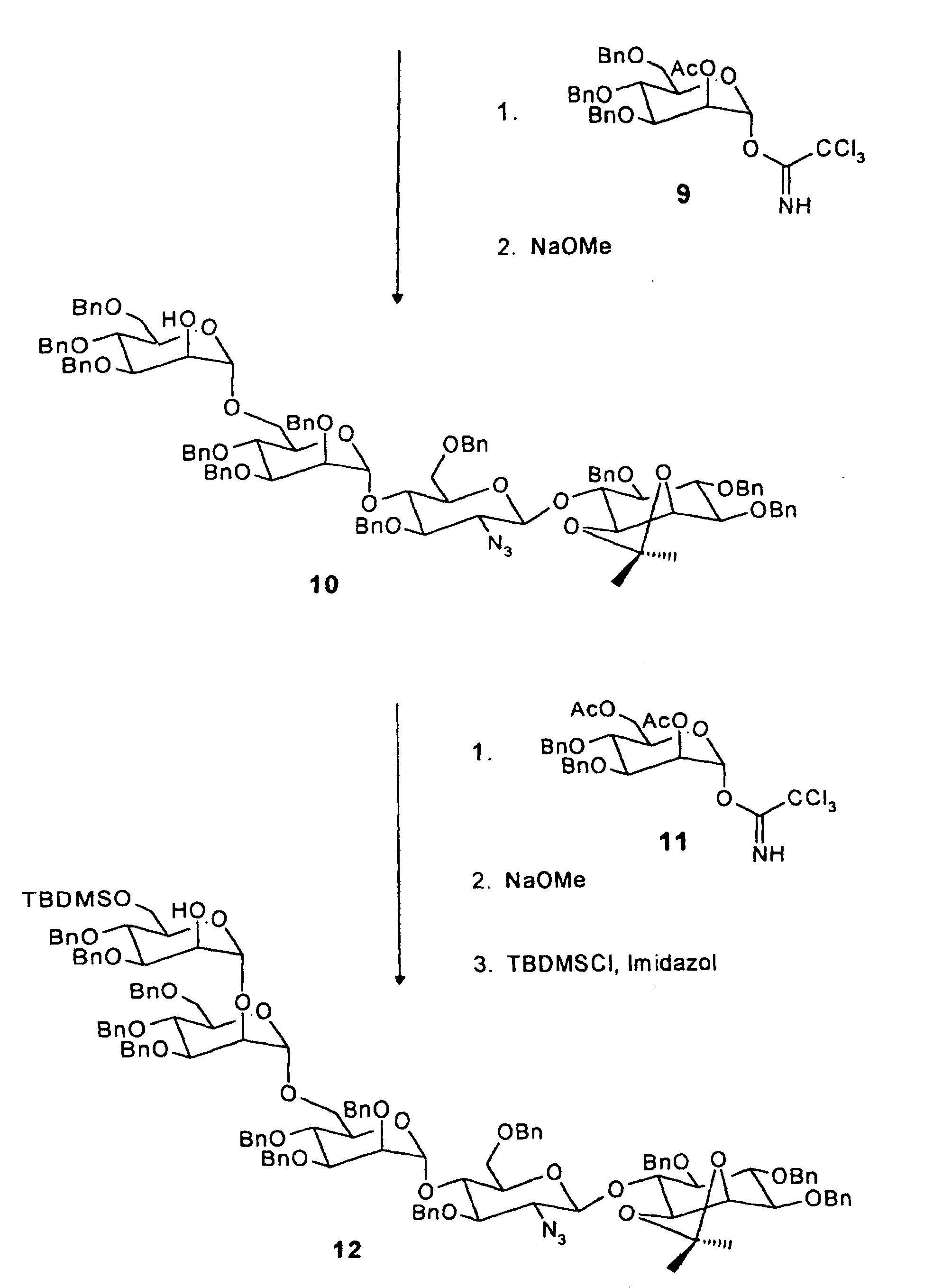

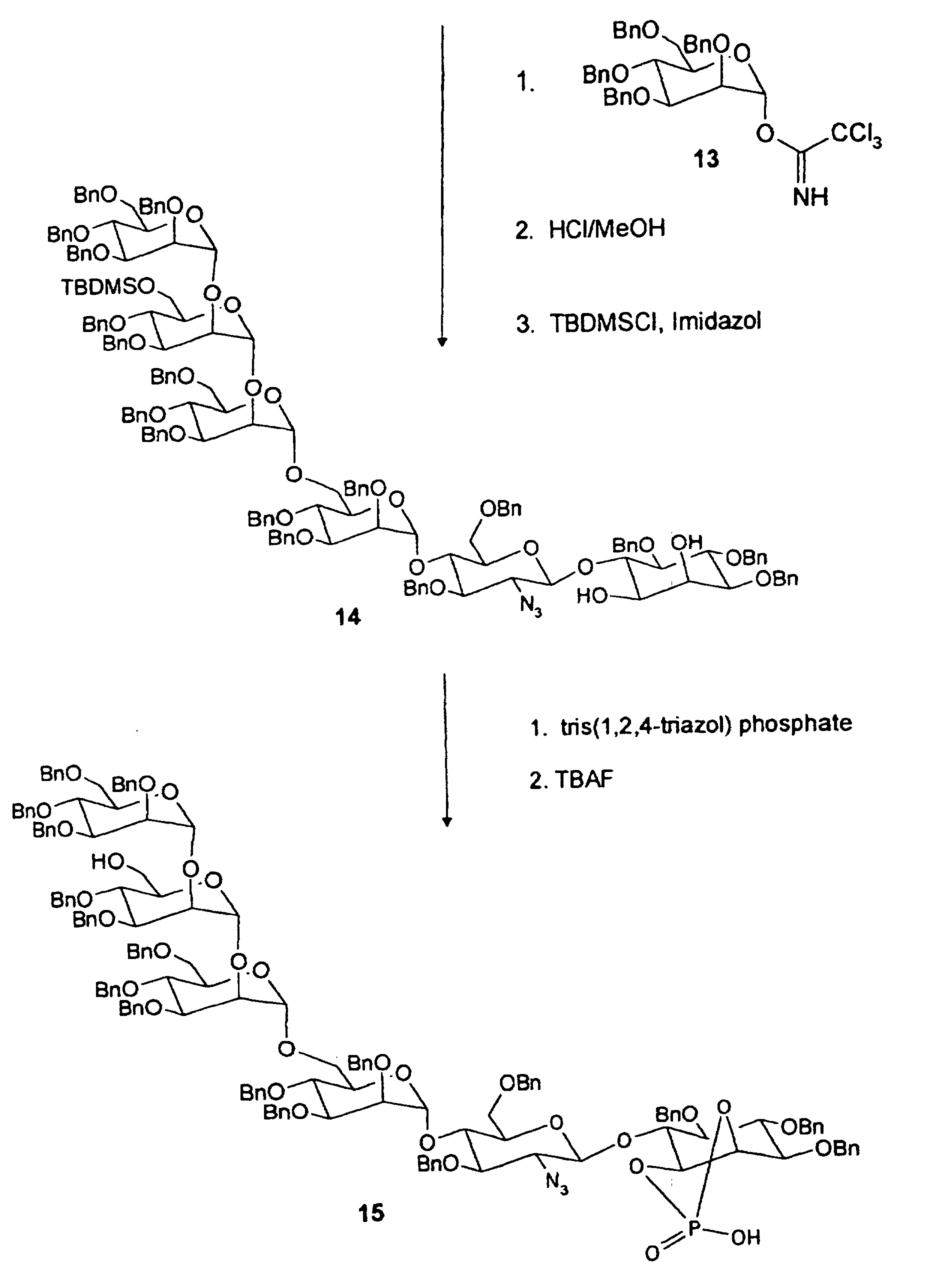

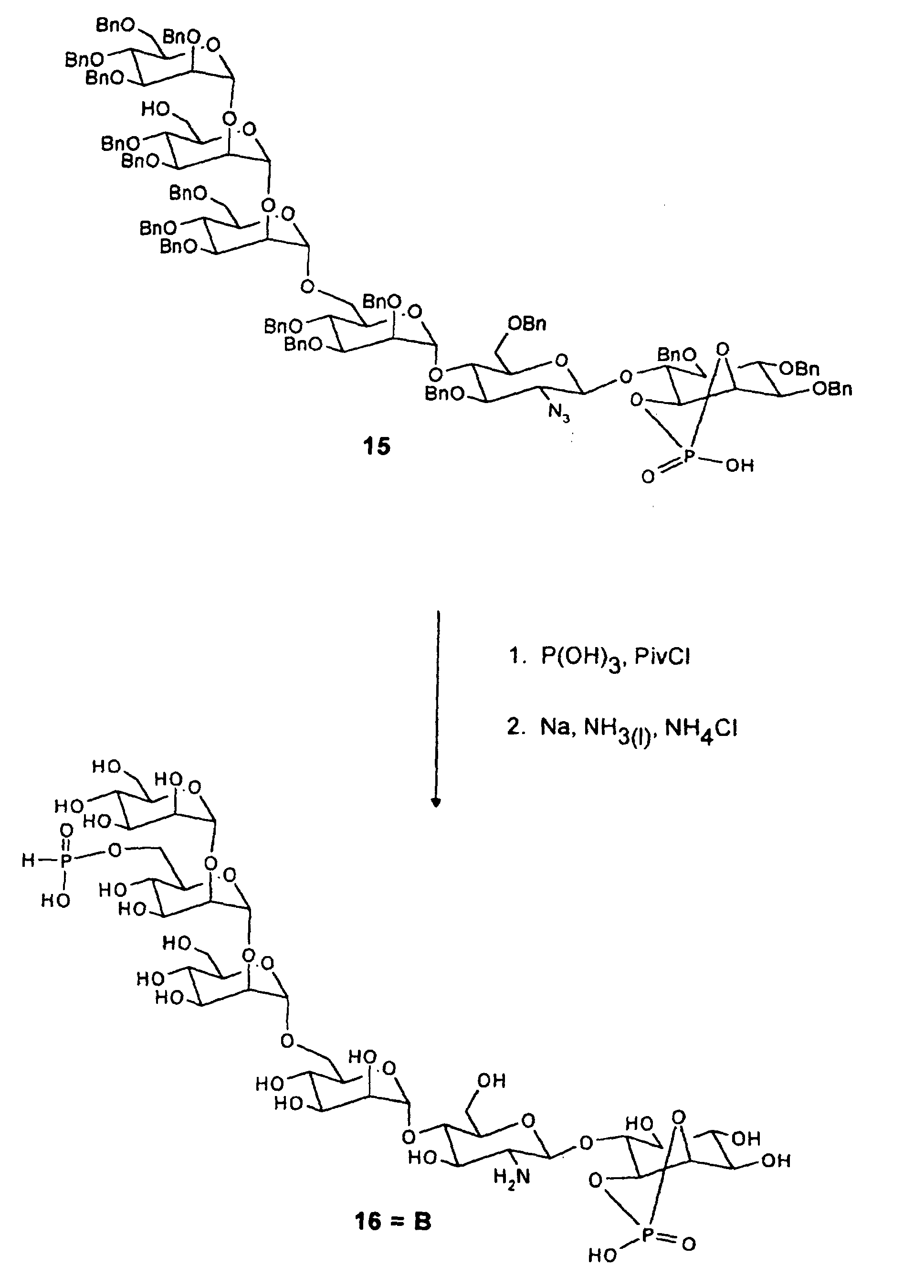

### Beispiel 2:

Die Verbindungen A, C, D, E, F, G, H, und J bis W (s. Tabelle 2) werden analog der Methoden gemäß Beispiel 1 hergestellt. Tabelle 1 zeigt die Strukturformeln, Summenformel und Massenspektrum der Verbindungen A, C, D, E, F, G, H und I. Die Massenspektren der Verbindungen J bis W stimmen mit den theoretisch berechneten Werten überein.

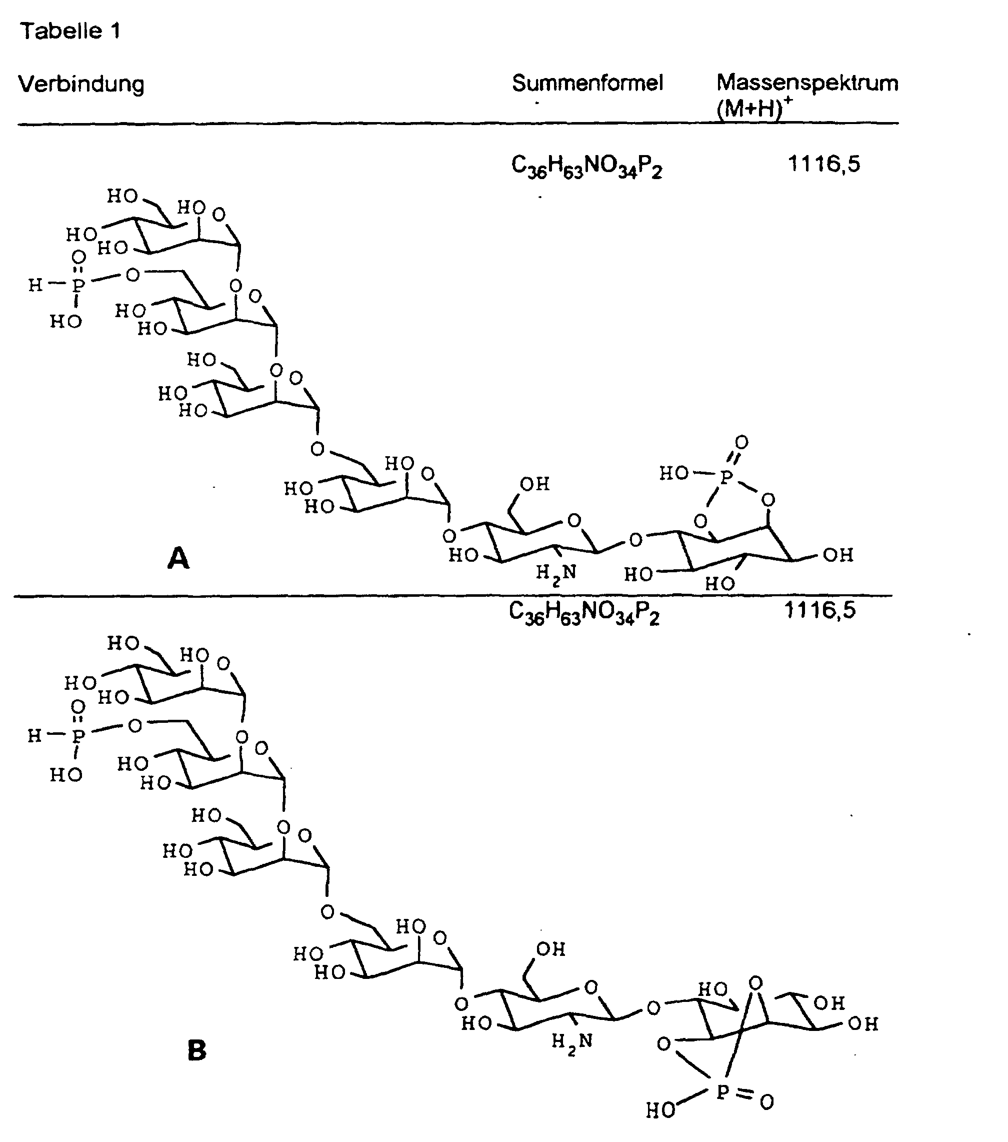

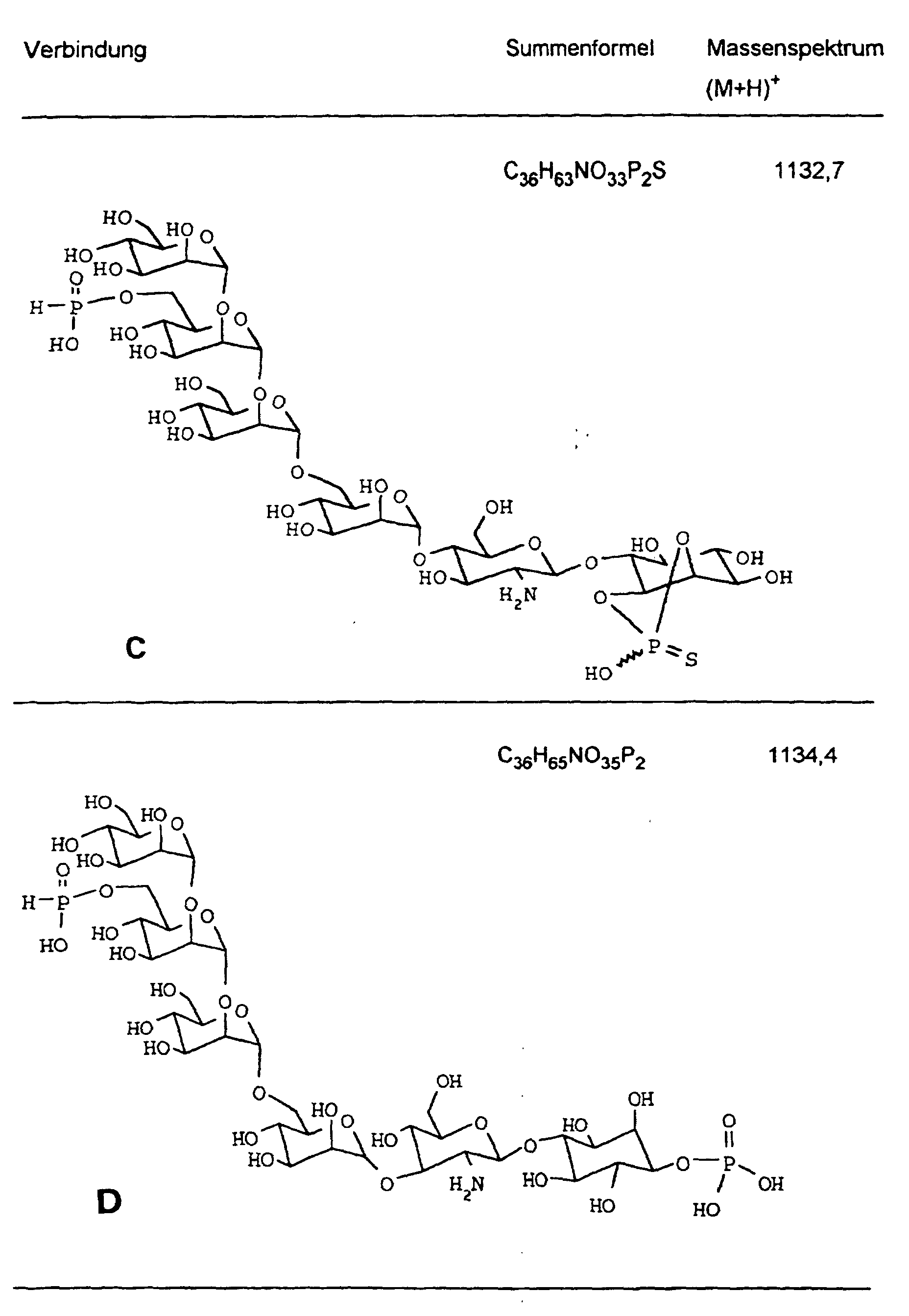

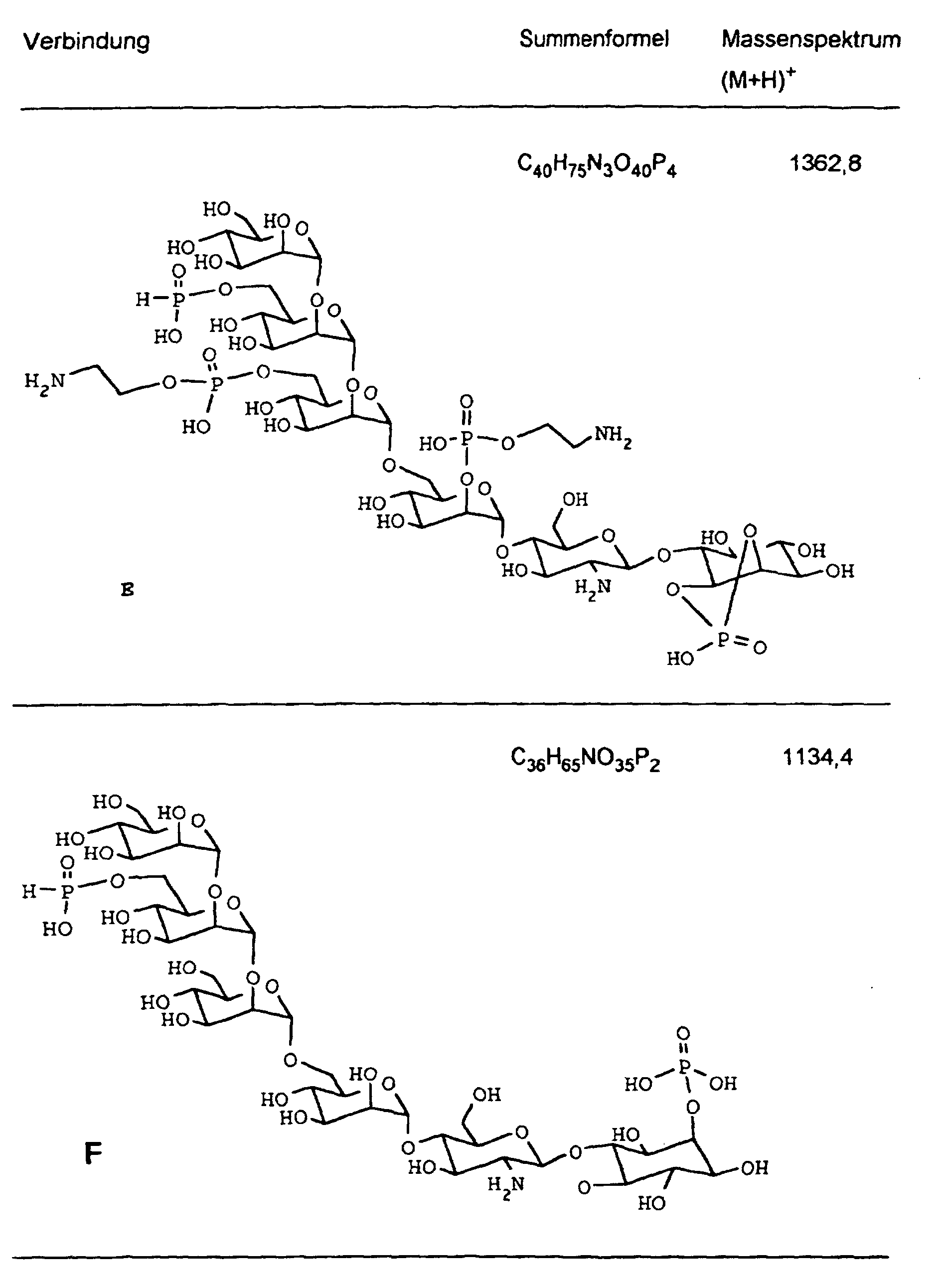

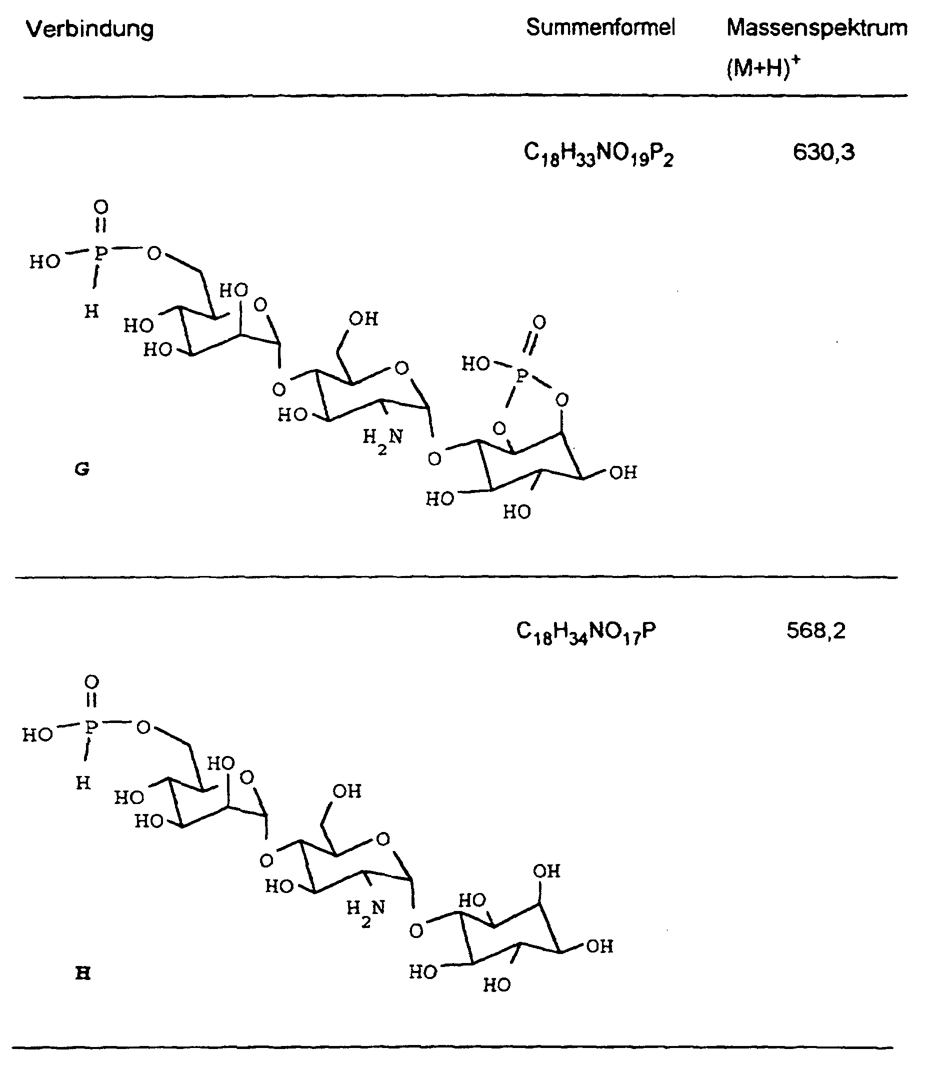

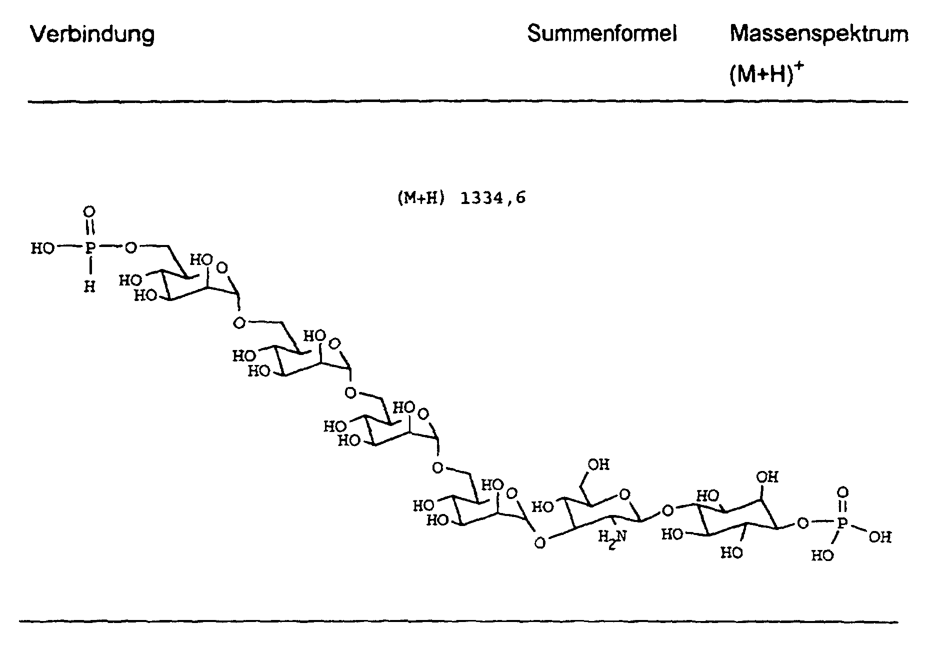

### Beispiel 3

### Pharmazeutische Wirksamkeit

Die biologische Aktivität der erfindungsgemäßen Verbindungen der Formel I wird anhand von isolierten Fettzellen aus der Ratte bestimmt.

Die Präparation von Fettzellen aus der Ratte erfolgte wie folgt:
Weißes Fettgewebe des Nebenhodens (Wistar-Ratte, 160 - 180 g, keine Futterbeschränkung) wird mit Kollagenase verdaut und die entstandenen vereinzelten Fettzellen werden über Filtration von unverdautem Gewebe abgetrennt und durch Flotation mehrmals mit Krebs-Ringer-Henseleit-Puffer (KRH-Puffer) gewaschen.

### A) Lipogenese

Dieser Test bestimmt die insulinstimulierbare Umwandlung von Glucose in Toluollösliche Produkte (Triglyzeride, Phospholipide, Fettsäuren), welche den Glucose-Transport und die Triglyzerid (Glycerin-3-P-Synthese, Veresterung)- /Phospholipid/Fettsäure-Synthese einschließlich der Insulin-Signalübertragungskaskade erfordert. Bei einer Glukosekonzentration von 2,5 mM im Test ist die Veresterung (und nicht die Glyzerin-3-P-Synthese einschließlich Glukosetransport) für die Stimulation der Lipogenese geschwindigkeitsbestimmend.

200 µl (3 x 10⁵ Zellen/ml) Ratten-Fettzellen in KRH-Puffer werden mit 100 µl D-[3-³H]Glucose (25 mM, 0,4 µCi) in Gegenwart oder Abwesenheit von Insulin (10 ng/ml) oder erfindungsgemäßer Verbindung der Formel I für 90 min bei 37°C inkubiert (Endvolumen 1 ml). Durch Zugabe eines Toluol-löslichen Szintillationscocktails (10 ml) werden die Zellen aufgeschlossen und die Lipide von wasserlöslichen Produkten und dem Inkubationsmedium abgetrennt. Nach Phasentrennung wird die in Lipidprodukte inkorporierte Radioaktivität durch Szintillationsmessung direkt ohne Entfernung der wäßrigen Phase bestimmt ([³H]Lipid [dpm*10⁻³]). Von dieser Radioaktivität wird ein Kontrollwert (Inkubation unter identischen Bedingungen jedoch ohne Zellen) subtrahiert. Die Lipogeneserate ist bis 120 min linear. Der maximale Stimulationsfaktor - dies ist das Verhältnis vom Inkubationsergebnis mit Insulin zum Inkubationsergebnis ohne Insulin - wird auf 100 % gesetzt. Die Prozentangaben unter "%Ins_{max"} sind Teile des so definierten maximalen Stimulationsfaktors. Der Begriff EC₅₀ gibt die Konzentration der Verbindung der Formel I an, bei der 50% der durch die jeweilige Verbindung der Formel zu erreichenden maximalen Stimulation zu beobachten ist.

### B) Glucosetransport

Rattenfettzellen in 100 µl KRH-Puffer (Titer 5 x 10⁵ Zellen/ml) werden mit Insulin oder mit der erfindungsgemäßen Verbindung der Formel I für 15 min bei 37°C unter leichtem Schütteln inkubiert. Nach Zugabe von 50 µl 2-[³H]Deoxyglukose (0,3 mM, 0,2 µCi) wird die Inkubation bei Raumtemperatur fortgesetzt. Nach bestimmten Zeitpunkten (0-20 min) werden 100 µl des Testansatzes entnommen und in ein Reaktionsgefäß (Inhalt 400 µl) überführt, worin 250 µl Dinonylphthalat vorgelegt sind. Nach Zentrifugation (15 000 x g, 1 min) werden die Zellen auf der Ölschicht vom Inkubationsmedium unterhalb der Ölschicht durch Zerschneiden des Röhrchens auf Höhe der Ölschicht abgetrennt und in ein Szintillationsgefäß überführt. Nach Zugabe von 5 ml wasserlöslicher Szintillationsflüssigkeit wird die Radioaktivität bestimmt. Diese gesamte zellassoziierte Radioaktivität wird um passiv in die Zellen diffundierte und in den Zellzwischenräumen eingeschlossene [³H] Deoxyglucose durch Subtraktion eines Kontrollwerts (Inkubation in Gegenwart des Glukosetransporthemmers Cytochalasin B) korrigiert. Die initiale (stimulierte) Glukosetransportgeschwindigkeit ist bis 15 min linear. Der maximale Stimulationsfaktor - dies ist das Verhältnis vom Inkubationsergebnis mit Insulin zum Inkubationsergebnis ohne Insulin - wird auf 100 % gesetzt.

Die Begriffe "%Insₘₐₓ und " EC_{50"} sind wie unter A) Lipogenese definiert.

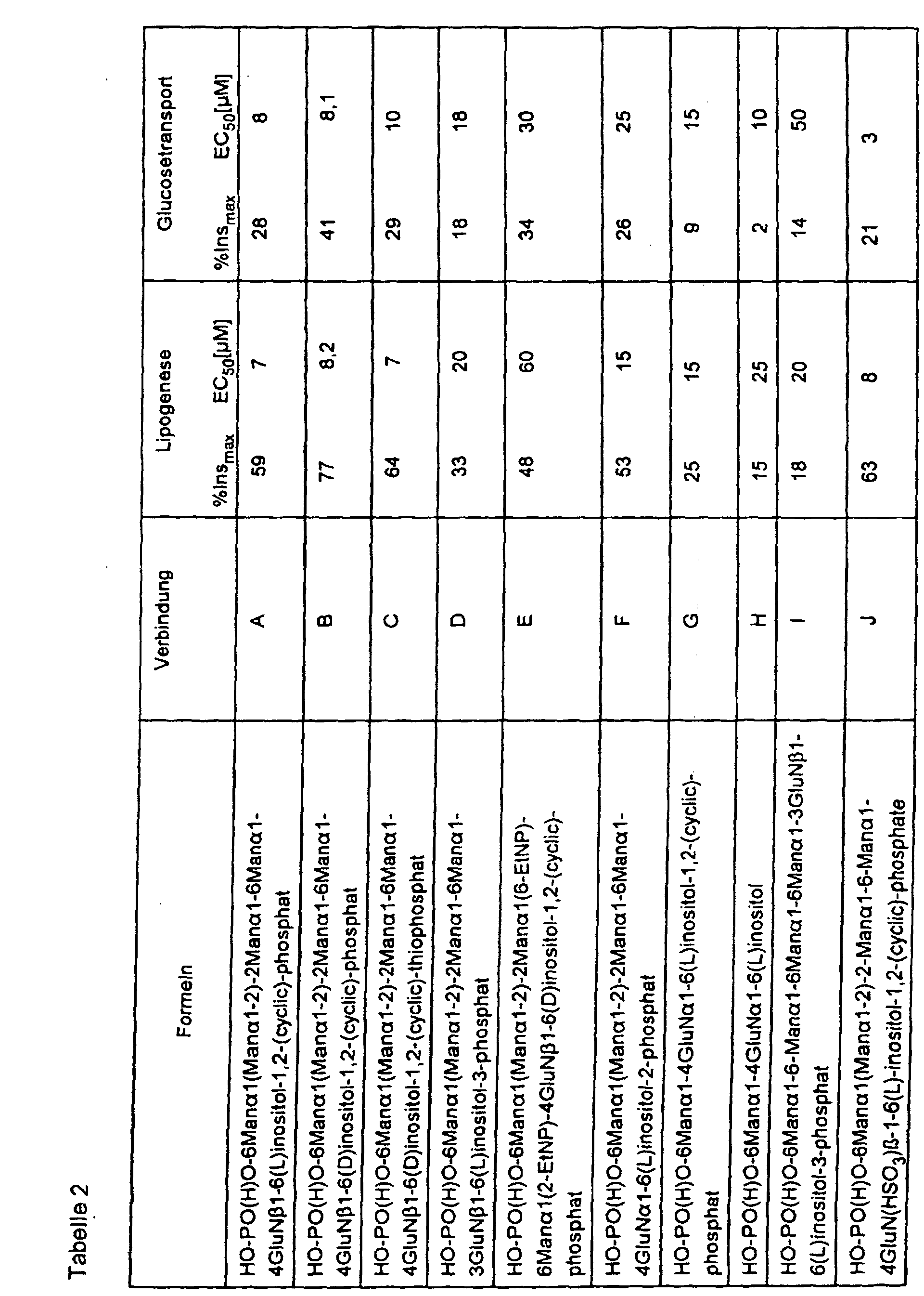

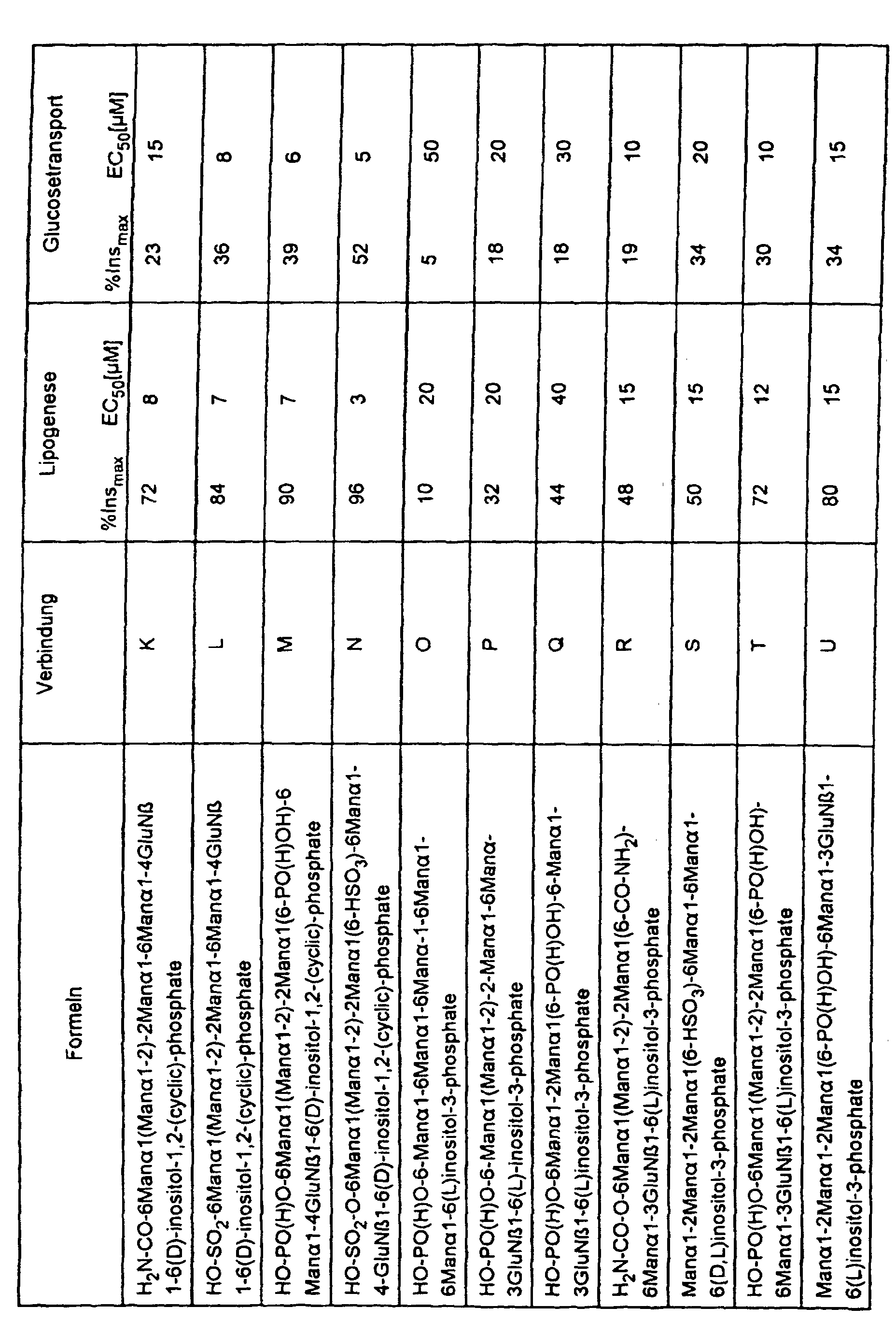

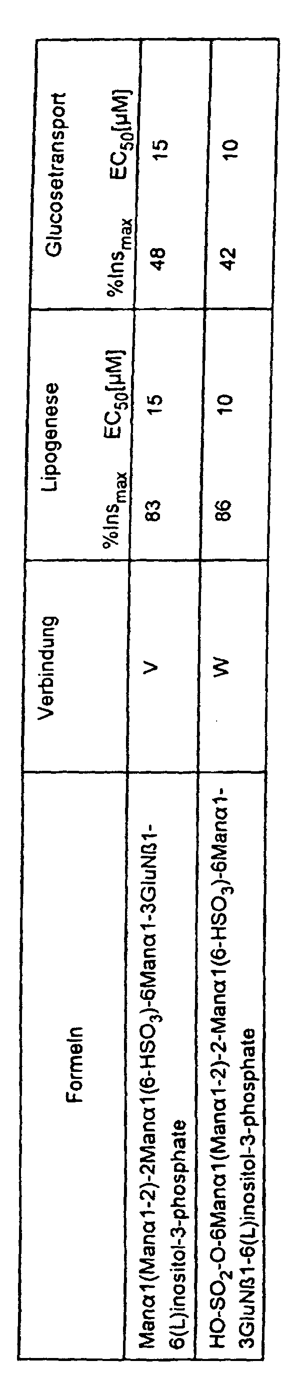

## Patentansprüche

1. Verbindung der Formel I
A-Z-R (I)
und/oder ein physiologisch verträgliches Salz der Verbindung der Formel I und/oder eine stereoisomere Form der Verbindung der Formel I, wobei
A für
1) H - P(O)(OH) -,
2) S(O)₂(OR¹) - oder
3) NH₂ - C(O) - steht,
Z für
1) 2 bis 6 Zuckerreste aus der Gruppe
1.1 Mannose,
1.2 Glucose,
1.3 Gluconsäure,
1.4 Galactonsäure,
1.5 Mannonsäure,
1.6 Glucosamin,
1.7 Fructose oder
1.8 Galaktose stammen,
2) 2 bis 6 Zuckerreste aus der Gruppe definiert unter Z 1.1 bis 1.8 stammen und ein- bis sechsfach unabhängig voneinander substituiert sind durch
2.1 Methyl,
2.2 Mannose,
2.3 Glucosamin,
2.4 Dimannose oder
2.5 Mannose-Glucosamin und die glykosidische Bindung der beiden Zucker Mannose und Glucosamin zwischen den C-Atomen 1-3, 1-2 oder 1-6 der beiden Zucker liegt, und
R für
1) Inositol,
2) Inositolphosphat,
3) Inositolthiophosphat,
4) Inositolcyclothiophosphat oder
5) Inositolcyclophosphat steht.

2. Verbindung der Formel I nach Anspruch 1, **dadurch gekennzeichnet, daß**
A für H - P(O)(OH) - steht,
Z für
1) 2 bis 4 Zuckerreste aus der Gruppe
1.1 Mannose oder
1.2 Glucosamin stammen oder
2) 2 bis 4 Zuckerreste aus der Gruppe
2.1 Mannose oder
2.2 Glucosamin stammen, einfach substituiert durch Mannose steht und
R für
1) Inositol,
2) Inositolphosphat oder
3) Inositolcyclophosphat steht.

3. Verfahren zur Herstellung einer Verbindung der Formel I nach einem oder mehreren der Ansprüche 1 oder 2, **dadurch gekennzeichnet, daß** man das Inositolglykan stufenweise aus geschützten Zucker- und Inositol-Vorstufen aufbaut, anschließend den Rest A anfügt und von der erhaltenen Verbindung eine oder mehrere zum Schutz anderer Funktionen temporär eingeführte Schutzgruppen abspaltet und die so gewonnene Verbindung der Formel I gegebenenfalls in ihr physiologisch verträgliches Salz überführt.

4. Pharmazeutische Zubereitung, **gekennzeichnet durch** einen wirksamen Gehalt an mindestens einer Verbindung der Formel I nach einem oder mehreren der Ansprüche 1 oder 2 in gelöster, amorpher oder kristalliner Form.

5. Pharmazeutische Zubereitung nach Anspruch 4, **gekennzeichnet durch** einen Gehalt an mindestens einem modifizierten oder unmodifizierten Insulin oder Insulinderivat.

6. Verfahren zur Herstellung einer pharmazeutischen Zubereitung nach Anspruch 4 oder 5, **dadurch gekennzeichnet, daß** man mindestens eine Verbindung der Formel I, mit einem physiologisch annehmbaren Träger sowie gegebenenfalls geeignete Zusatz- und/oder Hilfsstoffen in eine geeignete Darreichungsform bringt.

7. Verfahren nach Anspruch 6, **dadurch gekennzeichnet, daß** man mindestens ein modifiziertes oder unmodifiziertes Insulin oder Insulinderivat zusetzt.

8. Verwendung von mindestens einer Verbindung der Formel I nach einem oder mehreren der Ansprüche 1 oder 2 oder erhalten gemäß dem Verfahren nach Anspruch 3 zur Herstellung einer pharmazeutischen Zubereitung zur Behandlung des Diabetes mellitus oder nicht insulinabhängiger Diabetes.

## Claims

1. A compound of the formula I
A-Z-R (I)
and/or a physiologically tolerable salt of the compound of the formula I and/or a stereoisomeric form of the compound of the formula I, where
A is
1) H - P(O)(OH) -,
2) S(O)₂(OR¹) - or
3) NH₂ - C(O) -
Z is
1) 2 to 6 sugar radicals which originate from the group consisting of
1.1 mannose,
1.2 glucose,
1.3 gluconic acid,
1.4 galactonic acid,
1.5 mannonic acid,
1.6 glucosamine,
1.7 fructose or
1.8 galactose,
2) 2 to 6 sugar radicals which originate from the group defined under Z1.1 to 1.8 and are mono- to hexasubstituted independently of one another by
2.1 methyl,
2.2 mannose,
2.3 glucosamine,
2.4 dimannose or
2.5 mannose-glucosamine and the glycosidic bond of the two sugars mannose and glucosamine is between the carbon atoms 1-3, 1-2 or 1-6 of the two sugars, and
R is
1) inositol,
2) inositol phosphate,
3) inositol thiophosphate,
4) inositol cyclothiophosphate or
5) inositol cyclophosphate.

2. A compound of the formula I as claimed in claim 1, wherein
A is H - P(O)(OH) -,
Z is
1) 2 to 4 sugar radicals which originate from the group consisting of
1.1 mannose or
1.2 glucosamine or
2) 2 to 4 sugar radicals which originate from the group consisting of
2.1 mannose or
2.2 glucosamine, monosubstituted by mannose and
R is
1) inositol,
2) inositol phosphate or
3) inositol cyclophosphate.

3. A process for the preparation of a compound of the formula I as claimed in one or more of claims 1 or 2, which comprises synthesizing the inositolglycan stepwise from protected sugar and inositol precursors, then adding the radical A and removing from the compound obtained one or more protective groups introduced temporarily for the protection of other functions and converting the compound of the formula I thus obtained, if appropriate, into its physiologically tolerable salt.

4. A pharmaceutical preparation, comprising an efficacious amount of at least one compound of the formula I as claimed in one or more of claims 1 or 2 in dissolved, amorphous or crystalline form.

5. A pharmaceutical preparation as claimed in claim 4, comprising an amount of at least one modified or unmodified insulin or insulin derivative.

6. A process for the production of a pharmaceutical preparation as claimed in claim 4 or 5, which comprises bringing at least one compound of the formula I into a suitable administration form using a physiologically acceptable excipient and also, if appropriate, suitable additives and/or auxiliaries.

7. The process as claimed in claim 6, wherein at least one modified or unmodified insulin or insulin derivative is added.

8. The use of at least one compound of the formula I as claimed in one or more of claims 1 or 2 or obtained according to the process as claimed in claim 3 for the production of a pharmaceutical preparation for the treatment of diabetes mellitus or non insulin-dependent diabetes.

## Revendications

1. Composé de formule I
A-Z-R (I)
et/ou sel physiologiquement acceptable du composé de formule I et/ou forme stéréo-isomère du composé de formule I, dans laquelle
A représente 1) H-P(O)(OH)-,
2) S(O)₂(OR¹)- ou
3) NH₂-C(O)-,
Z représente
1) 2 à 6 restes glucidiques choisis dans le groupe
1.1 mannose,
1.2 glucose,
1.3 acide gluconique,
1.4 acide galactonique,
1.5 acide mannonique,
1.6 glucosamine,
1.7 fructose et
1.8 galactose,
2) 2 à 6 restes glucidiques choisis dans le groupe défini en Z 1.1 à 1.8 et une à six fois substitués, indépendamment les uns des autres, par
2.1 méthyle,
2.2 mannose,
2.3 glucosamine,
2.4 dimannose ou
2.5 mannose-glucamine et la liaison glycosidique des deux sucres mannose et glucamine se trouve entre les atomes de carbone 1-3, 1-2 ou 1-6 des deux sucres, et
R représente
1) l'inositol,
2) le phosphate d'inositol,
3) le thiophosphate d'inositol,
4) le cyclothiophosphate d'inositol ou
5) le cyclophosphate d'inositol.

2. Composé de formule I selon la revendication 1, **caractérisé en ce que**
A représente H-P(O)(OH)-,
Z représente
1) 2 à 4 restes glucidiques choisis dans le groupe
1.1 mannose et
1.2 glucosamine ou
2) 2 à 4 restes glucidiques choisis dans le groupe
2.1 mannose et
2.2 glucosamine, monosubstituée par le mannose et
R représente
1) l'inositol,
2) le phosphate d'inositol ou,
3) le cyclophosphate d'inositol.

3. Procédé pour la préparation d'un composé de formule I selon une ou plusieurs des revendications 1 et 2, **caractérisé en ce qu'**on synthétise par étapes l'inositolglycane à partir de précurseurs sucre et inositol protégés, on ajoute ensuite le reste A et on élimine du composé obtenu un ou plusieurs groupes protecteurs introduits temporairement pour la protection d'autres fonctions, et éventuellement on convertit le composé de formule I ainsi obtenu en son sel physiologiquement acceptable.

4. Préparation pharmaceutique, **caractérisée par** une teneur efficace en au moins un composé de formule I selon une ou plusieurs des revendications 1 et 2, sous forme dissoute, amorphe ou cristalline.

5. Préparation pharmaceutique selon la revendication 4, **caractérisée par** un teneur en au moins une insuline ou un dérivé d'insuline, modifié(e) ou non modifié(e).

6. Procédé pour la fabrication d'une préparation pharmaceutique selon la revendication 4 ou 5, **caractérisé en ce qu'**on met sous une forme d'administration appropriée au moins un composé de formule I avec un véhicule physiologiquement acceptable ainsi qu'éventuellement des additifs et/ou adjuvants appropriés.

7. Procédé selon la revendication 6, **caractérisé en ce qu'**on ajoute au moins une insuline ou un dérivé d'insuline modifié(e) ou non modifié(e).

8. Utilisation d'au moins un composé de formule I selon une ou plusieurs des revendications 1 et 2 ou obtenu conformément au procédé selon la revendication 3, pour la fabrication d'une préparation pharmaceutique destinée au traitement du diabète sucré ou du diabète non insulino-dépendant.
